# EUROPEAN PATENT SPECIFICATION

(11) **EP 2 956 078 B1**
(45) Date of publication and mention of the grant of the patent: **06.04.2022**
(21) Application number: 13874996.5
(22) Date of filing: 13.02.2013
(51) Int. Cl.: A61B 18/20, A61N 5/067, A61B 18/00, A61B 90/00

(54) **ENCLOSED LASER MEDICAL DEVICE/SYSTEM**
MEDIZINISCHE LASERVORRICHTUNG/SYSTEM IN EINEM GEHÄUSE
DISPOSITIF/SYSTÈME MÉDICAL À LASER CONFINÉ

(43) Date of publication of application: 23.12.2015
(73) Proprietor: biolitec Unternehmensbeteiligungs II AG, 1030 Vienna (AT)
(72) Inventor: NEUBERGER, Wolfgang, Dubai (AE)
(74) Representative: Herrmann, Franz
(86) International application number: PCT/US2013/025915
(87) International publication number: WO 2014/126558

(56) References cited:
- WO-A1-2009/023801
- US-A- 4 757 515
- US-A1- 2007 060 984
- US-A1- 2008 077 170
- US-A1- 2008 077 200
- US-A1- 2008 203 280
- US-A1- 2010 106 146

## Description

### Background of the Invention

### 1. Field of the invention

The present invention relates to minimally invasive medical treatment systems and in particular, to medical treatments/systems using self-contained energy emitting devices and conveying means to provide safe tools in medical environments.

### 2. Prior Art Disclosure Statement

Laser systems can operate as beneficial and effective medical instruments. They allow specific treatment to be administered with minimal invasiveness. Laser treatments are frequently preferred by those skilled in the art in different applications. There is an increasing number of medical applications involving the use of laser devices in a wide range of specialized disciplines such as angiology, proctology, otolaryngology, urology, gynecology and aesthetics. Worth specific mention are treatment of insufficient veins, benign hyperplasic prostate (BPH), hemorrhoids, ulcers, fistulas, calculi, and ear, nose and throat (ENT) disorders just to name a few.

Minimally invasive laser treatments have been improved due to new diode laser systems. Diode laser systems are greatly advantageous in comparison with other existing laser source technologies such as CO2, holmium:YAG, pulsed-dye, Nd:YAG or KTP laser sources, in that they provide higher output, at reduced dimensions and weight. They also have simpler and smaller air cooling systems. Moreover, being capable of integration with optical fibers, they have a high reliability and do not need alignment. In summary, they provide great efficiency and robustness.

Consequently, there are also an increasing number of different scenarios in which laser devices are needed, ranging from the physician's office to high tech operating rooms. Moreover, many single laser devices are unspecific enough so that one same device is optimal for more than one type of treatment. In many cases, the same laser device can be used in the operating room for aesthetic surgery; within a well-equipped vascular surgeon's office; or in a simpler otolaryngologist's office. However, when all of these scenarios exist in one healthcare center, there is at least one of these medical devices assigned to each treatment application, since their size, weight and complexity may not justify a logistics system to rotate one device among the different areas. Furthermore, the size of existing equipment requires a certain storage space that may not be available in every health unit in which it is used. Additionally, the number of cables involved in connecting equipment make using the device complex and uncomfortable and in some cases dangerous. Finally, in some cases, equipment belongs to physicians, not to the health care center where they work, and because of size and weight it is difficult for them to take equipment with them to, for example to other offices or healthcare centers, where the physician also practices.

Thus, medical device designers have made attempts to address some of aforementioned drawbacks. Below are some examples of such attempts.

In US Patent Publication 2008/0077198 by Webb et al, a handheld self-contained nerve-stimulation device using light to provide a source of stimulation on one or more nerve fibers is disclosed. Stimulation is provided through a device wherein a laser or LED light source is mounted to the hand piece. Light is passed from the light source through optical tip to stimulate nerves. The hand piece contains a self-contained power source, such as batteries. US Application 2010/0106146 by Boitor et al. discloses a handheld portable diode laser surgical device that includes a power supply, a laser diode, integral control interface and display, and a multicomponent sterile, disposable tip apparatus featuring assembly for alignment of a self-contained optical fiber to the surgical device, and releasable locking assembly between the tip apparatus and surgical device. An embodiment includes wireless foot pedal on/off control and a dock providing sterile, antiseptic recharging environment.

US Patent Application 2009/0275930 by Di Sessa et al. discloses a multicomponent sterile, disposable tip apparatus for laser surgical devices. It features assembly for alignment of a self-contained optical fiber to the surgical device and releasable locking assembly between the tip and device. US Patent 6,724,958 by German et al. discloses an invention that is a self-contained handheld surgical laser that provides surgeons with the optimal laser beam parameters for laser tissue soldering. The laser is incorporated in a battery powered self- contained handheld apparatus with a roughly uniform distribution of light intensity across the beam. The laser apparatus provides laser energy at a wavelength absorbed by the chromophore utilized in the laser solder with optimum parameters for tissue soldering.

US patent 4,757,515 discloses a beam generator that can be inserted into a casing, wherein the beam generator is held within the casing by two O-rings disposed around both ends of the beam generator. The casing can be made water tight and contamination free by sealing its output end via an optical window or optical lens and sealing a cable into the rear end.

US patent application 2008/0203280 relates to a system comprising a laser module from which a trunk optical fiber extends to a handpiece. The handpiece may be provided with a sterile output tip that can be replaced if necessary.

Mentioned, existing prior art possesses several limitations. First, these are very low power units. Output energy emitted from devices is insufficient or ineffective for several common medical applications. Secondly, laser source is not presented as sterile. Additionally, the small-sized prior art does not consider itself with easy maneuverability, an important issue in many delicate medical applications. Finally, none of mentioned devices gather all the features described above in a single sterile device, namely, small size, low cost, cable-less, and easy to handle. Consequently, formerly disclosed compact-sized devices are difficult to maneuver, involve discomfort for the physician and a potential hazard for the patient.

There is thus, a need for a safe, compact, low cost, disposable medical laser treatment device/system that is versatile and easy to maneuver. The present invention addresses these needs.

### Objectives and Brief Summary of the Invention

It is an objective of the present invention to provide a device for treatment of different medical conditions.

It is another objective of the present invention to treat several types of medical conditions by using a single localized, directed energy source and conveying means.

It is yet another objective of the present invention to provide a portable and versatile sterile laser medical device/system.

It is still another objective of the present invention to provide medical laser device that is safe and easily maneuverable.

These objectives are achieved by devices having the features of the independent claim. Advantageous embodiments and refinements are specified in claims dependent thereon.

Briefly stated, a fiber optic medical device/system is presented, comprising means to connect and enclose a laser source to ensure sterility and safe operation. Device includes a low cost, single use system enclosed and sterilized including laser source and with the fiber attached in a sterile tube. A preferred embodiment comprises hermetically sealed laser unit with a simple connection system to a properly shaped short fiber optic, where, after joining, unit comprises a hand piece and fiber for direct insertion to treat targeted tissue. Laser handle is aseptically packaged. In other embodiments, fiber is proximally terminated in solid funnel-shaped end to provide unique, directional keyed junction with laser module. In another embodiment, unit includes fiber proximally joined to it, where a laser module can be added away from the fiber end, so that laser does not need to be sterilized but entire unit is safe for medical use in sterile fields. Enclosure also serves as a grip for pulling and/or manipulating device. In another preferred embodiment, laser unit is attached to a rigid delivery system for laparoscopic procedures.

The above and other objects, features and advantages of the present invention will become apparent from the following description read in conjunction with the accompanying drawings (in which like reference numbers in different drawings designate the same elements).

### Brief Description of Figures

FIG. 1 depicts an embodiment describing main components of the system disclosed.
FIG. 2 shows preferred connection systems between laser device and conveying means.
FIG. 3 depicts a sketch of a preferred embodiment of present invention.
FIG. 4 shows another preferred embodiment with a complete disposable unit with rigid delivery end.

### Detailed Description of Preferred Embodiments

The present invention addresses prior art disadvantages by providing a portable, cable-less, disposable laser device for carrying out a number of different medical treatments while assuring sterility.

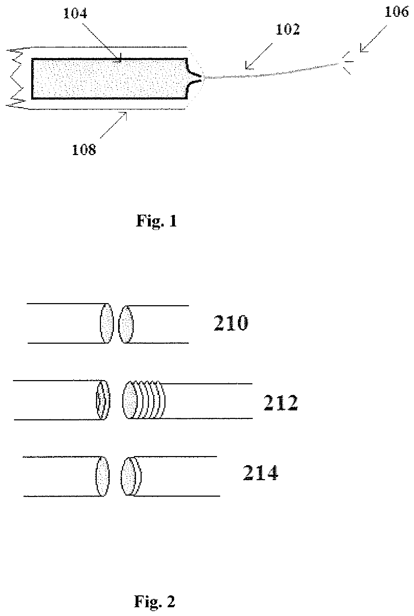

In a preferred embodiment, depicted in **FIG. 1****,** system comprises a fiber optic medical treatment device **102** attached to source **104,** which is kept within sterile enclosure **108.** Optical fiber's firing end **106** emits radiation at target tissue by means of a variety of possible radiation patterns according to firing end including but not limited to off-axis firing end, a side-firing distal end, a radial emitting end or a direct emitting end. When laser radiation is used to apply energy to the vessel, different wavelengths can be chosen. Laser wavelength is chosen, in the present case, according to the desired penetration depth in tissue and desired effect in tissue. Radiofrequency, microwave, thermal and other energy sources may be used to reliably and controllably perform the task and the method described, provided suitable enhancers and/or imaging means as described are used.

In a preferred embodiment, system comprises hermetically sealed laser unit with simple hook up system to an optical fiber. Hook up systems, as shown in **FIG. 2** include but are not limited to plug-socket (male-female), **210,** screw-on **212,** or snap-on **214** connectors. Such hook up systems can be stereo-specific to provide a specific orientation of the fiber distal tip relative to hand piece. Once fiber optic is connected, unit controls a hand piece and fiber for direct insertion into target tissue. Laser handle is aseptically packaged. In preferred embodiments, fiber is proximally terminated in solid funnel-shaped end to provide unique directional keyed junction with laser module. In another preferred embodiment, unit includes fiber proximally joined to it, where a laser module can be added away from the fiber end. This way laser source/emitter does not need to be sterilized and entire unit is safe for medical use in sterile fields. In another embodiment, outer contour of laser module has a grooved pattern to permit easy grip. This way, enclosure also serves as a grip for making maneuvers with device/system as for example a pull-back movement during insufficient vein treatments; twisting motion for urologic treatments or back-and-forth movements in liposuction techniques.

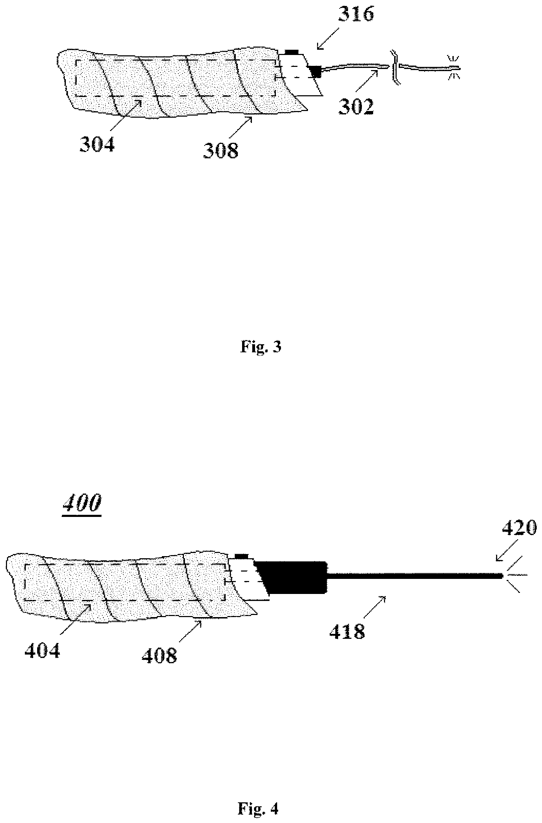

An example of a preferred embodiment is a small-sized device with an ergonomic handgrip for portable handheld applications. **FIG 3** shows a sketch of preferred embodiment. Energy source **304** is a 5 Watt 1470 nm battery driven diode laser that conveys energy through a 30-40 cm long optical fiber **302.** Alternatively, for higher energy applications, energy source **304** is an electrically driven 20-Watt diode laser. Enclosure **308** also plays the role of a handgrip. System includes an RFID technology system 316 to identify the laser/fiber package and/or permit activation for specific parameters such as power level, energy and density. This eliminates the need for cables that make handling medical devices unwieldy, uncomfortable and sometimes even dangerous.

In another preferred embodiment, as depicted in **Fig. 4****,** a complete disposable unit **400** includes a laser source **404** enclosed within ergonomic handgrip **408** attached to a rigid conveying means **418** and delivery end **420** for more complex surgical procedures such as laparoscopic procedures. In another embodiment, such unit is handled via remote control for complex robotic surgeries.

Embodiments in this invention present several advantages. One advantage is its potentially small size. Another advantage is its versatility for several different application configurations. It is thus, a useful tool for health care settings that carry out various different laser treatments. This possibility allows the health care center to be more efficient and to reduce costs. Additionally, because it can be controlled by RFID technology and system is battery operated, cables are not needed. This contributes also to its versatility. Finally, disclosed invention is low in cost and is therefore feasible for consideration as a practical, compact, low cost, disposable device in many health care settings, such as in different hospital care units and specific independent treatment offices.

## Claims

1. A fiber optic medical treatment device, which is cable-less, portable and disposable, comprising:
a battery powered laser energy source (104, 304);
an enclosure (108, 308), which keeps and hermetically seals the battery powered laser energy source (104, 304) to ensure sterility and provide safe operation;
an optical fiber (302) having a distal firing end (106) located outside the enclosure (108, 308); and
a connection system for connecting the battery powered laser energy source (104, 304) with the optical fiber (302).

2. The fiber optical medical treatment device according to claim 1,
wherein the connection system is plug-socket connector (210), a screw-on connector (212) or a snap-on connector (214).

3. The fiber optic medical treatment device according to claim 1 or 2,
wherein in the connection system to said optical fiber (302) is stereo-specific to provide a specific orientation of the fiber distal tip to the enclosure.

4. The fiber optical medical treatment device according to any one of claims 1 to 3, wherein said optical fiber (302) comprises a funnel-shaped proximal end for a directional keyed junction with said laser energy source (104, 304).

5. The fiber optical medical treatment device according to any one of claims 1 to 4, wherein said enclosure (308) serves as a handgrip for manipulating said device.

6. The fiber optical medical treatment device according to any one of claims 1 to 5, further comprising RFID technology to identify a laser or fiber package.

7. The fiber optical medical treatment device according to any one of claims 1 to 6, further comprising RFID technology to control radiation or operating parameters.

## Patentansprüche

1. Faseroptische medizinische Behandlungsvorrichtung, die kabellos, tragbar und wegwerfbar ist, mit:
einer batteriebetriebenen Laserenergiequelle (104, 304);
einem Gehäuse (108, 308), das die batteriebetriebene Laserenergiequelle (104, 304) aufnimmt und hermetisch abdichtet, um Sterilität und einen sicheren Betrieb zu gewährleisten;
einer optischen Faser (302) mit einem distalen Abstrahlsende (106), das sich außerhalb des Gehäuses (108, 308) befindet; und
einem Verbindungssystem zum Verbinden der batteriebetriebenen Laserenergiequelle (104, 304) mit der optischen Faser (302).

2. Faseroptische medizinische Behandlungsvorrichtung nach Anspruch 1,
bei der das Verbindungssystem ein Steckverbinder (210), ein Schraubverbinder (212) oder ein Schnappverbinder (214) ist.

3. Faseroptische medizinische Behandlungsvorrichtung nach Anspruch 1 oder 2,
bei der das Verbindungssystem zur optischen Faser (302) stereospezifisch ist, um eine spezifische Ausrichtung der distalen Spitze der Faser zum Gehäuse zu gewährleisten.

4. Faseroptische medizinische Behandlungsvorrichtung nach einem der Ansprüche 1 bis 3,
bei der die optische Faser (302) ein trichterförmiges proximales Ende für eine gerichtete formschlüssige Verbindung mit der Laserenergiequelle (104, 304) aufweist.

5. Faseroptische medizinische Behandlungsvorrichtung nach einem der Ansprüche 1 bis 4,
bei der das Gehäuse (308) als Handgriff für die Handhabung der Vorrichtung dient.

6. Faseroptische medizinische Behandlungsvorrichtung nach einem der Ansprüche 1 bis 5,
die ferner RFID-Technologie zur Identifizierung eines Laser- oder Faserverpackung aufweist.

7. Faseroptische medizinische Behandlungsvorrichtung nach einem der Ansprüche 1 bis 6,
die ferner RFID-Technologie zur Steuerung der Strahlung oder der Betriebsparameter aufweist.

## Revendications

1. Dispositif de traitement médical à fibre optique, qui est sans fil, portable et jetable, comprenant :
une source d'énergie laser alimentée par piles (104, 304) ;
une enveloppe (108, 308) qui maintient et scelle hermétiquement la source d'énergie laser alimentée par piles (104, 304) pour garantir la stérilité et fournir un fonctionnement sûr ;
une fibre optique (302) ayant une extrémité de déclenchement distale (106) située en dehors de l'enveloppe (108, 308) ; et
un système de connexion pour connecter la source d'énergie laser alimentée par piles (104, 304) à la fibre optique (302).

2. Dispositif de traitement médical à fibre optique selon la revendication 1,
dans lequel le système de connexion est un connecteur fiche-prise (210), un connecteur à visser (212) ou un connecteur à enclenchement (214).

3. Dispositif de traitement médical à fibre optique selon la revendication 1 ou 2,
dans lequel le système de connexion à ladite fibre optique (302) est stéréospécifique pour fournir une orientation spécifique de la pointe distale de fibre vers l'enveloppe.

4. Dispositif de traitement médical à fibre optique selon l'une quelconque des revendications 1 à 3,
dans lequel ladite fibre optique (302) comprend une extrémité proximale en entonnoir pour une jonction clavetée directionnelle avec ladite source d'énergie laser (104, 304).

5. Dispositif de traitement médical à fibre optique selon l'une quelconque des revendications 1 à 4,
dans lequel ladite enveloppe (308) sert de poignée pour manipuler ledit dispositif.

6. Dispositif de traitement médical à fibre optique selon l'une quelconque des revendications 1 à 5,
comprenant en outre de la technologie RFID pour identifier une configuration de laser ou de fibre.

7. Dispositif de traitement médical à fibre optique selon l'une quelconque des revendications 1 à 6,
comprenant en outre de la technologie RFID pour commander des paramètres de rayonnement ou de fonctionnement.
